(19) Europäisches Patentamt
European Patent Office
Office européen des brevets

(11) Publication number: 0 151 528
B1

(12) EUROPEAN PATENT SPECIFICATION

(45) Date of publication of patent specification: 04.07.90

(51) Int. Cl.[5]: C 07 D 249/04, A 61 K 31/41
// C07C255/03, C07C255/65

(21) Application number: 85300520.5

(22) Date of filing: 25.01.85

# (54) 5-(Amino or substituted amino)-1,2,3-triazoles.

(30) Priority: 02.02.84 US 576302

(43) Date of publication of application:
14.08.85 Bulletin 85/33

(45) Publication of the grant of the patent:
04.07.90 Bulletin 90/27

(84) Designated Contracting States:
CH DE FR GB IT LI NL

(56) References cited:
EP-A-0 113 570
EP-A-0 114 347
US-A-3 577 553

IL FARMACO, EDIZINA SCIENTIFICA, vol. 35, no. 4, 1980, pages 298-307, Pavia, IT; A. DA SETTIMO et al.: "Synthesis and pharmacological activity of some 9-aryl-8-azapurine derivatives"

IL FARMACO, EDIZIONE SCIENTIFICA, vol. 35, no. 4, 1980, pages 308-323, Pavia, IT; A. DA SETTIMO et al.: "Synthesis and pharmacological activity of three new 9-aryl-8-azaadenine derivatives"

(73) Proprietor: MERCK & CO. INC.
126, East Lincoln Avenue P.O. Box 2000
Rahway New Jersey 07065-0900 (US)

(72) Inventor: Harris, Elbert E.
220 Linden Avenue
Westfield New Jersey 07090 (US)
Inventor: Tolman, Richard L.
29 Upper Warren Way
Warren New Jersey 07060 (US)

(74) Representative: Crampton, Keith John Allen et al
D YOUNG & CO 10 Staple Inn
London WC1V 7RD (GB)

(56) References cited:
IL FARMACO, EDIZIONE SCIENTIFICA, vol. 34, no. 3, 1979, pages 217-228, Pavia, IT; O. LIVI et al.: "Sintesi ed esame farmacologico de derivati 1,2,3-triazolici della naftaline, chinolina e piridina"

EP 0 151 528 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

(56) References cited:
JOURNAL OF THE CHEMICAL SOCIETY, no. 4, 1971, pages 706-713, Londen, GB; D.R. SUTHERLAND et al.: "The chemistry of polyazaheterocyclic compounds. Part IV. Dimroth rearrangements of 4-substituted 5-amino-1-phenyl-1,2,3-triazoles and a synthesis of v-triazolo4,5-drpyrimidines"

JOURNAL OF THE CHEMICAL SOCIETY, 1969, no. 1, pages 152-160, Londen, GB; A. ALBERT et al.: "1,2,3,4,6-penta-azaindenes (8-azapurines). Part V. A comparison of 1,2,3-triazoles and pyrimidines as intermediates for the preparation of 9-substituted 8-azapurines. Rearrangement of 6-mercapto-8-azapurines and of 4-aminotriazoles"

JOURNAL OF THE CHEMICAL SOCIETY, no. 17, 1974, pages 2030-2036, Londen, US; A. ALBERT et al.: "v-Triazolo4,5-drpyrimidines (8-azapurines). Part XV. Degradation by acid of ring N-alkylated derivatives of 6-amino-(and 1,6-dihydro-6-imino-)8-azapurines to N-alkylated 4-amino-1,2,3-triazole-5-carbox-amidines"

CHEMICAL ABSTRACTS, vol. 91, no. 24, 17th December 1979, page 27, no. 204219s, Columbus, Ohio, US; A. LUCACCHINE et al.: "Effects of substituted 1,2,3-triazoles on adenosine deaminase guanine deaminase and xanthine oxidase", & ITAL. J. BIOCHEM. 1979, 28(3), 194-206

**Description**

This invention relates to triazole compounds, their preparation, and their use.

Various 5-amino-4-substituted 1,2,3-triazoles with a 5-N-aryl or 1-aryl substituent are disclosed in Il Farmaco, Vol 34, No 3, 1979, pages 217—228, and Vol 35, No 4, 1980, pages 298—307 and 308—232; in J.C.S. (1971) No 4, pages 706—713, (1969) No 1, 152—160 and (1974) No 17, pages 2030—2036; and in Chem. Abs., Vol 91 No 24, 204219s.

Coccidiosis is a wide-spread poultry disease which is produced by infections of protozoa of the genus *Eimeria* which causes severe pathology in the intestines and caeca of poultry. Some of the most significant of these species are *e. tenella, E. acervulina, E. necatrix, E. brunetti, E. maxima, E. mitis, E. mivati, E. hagani* and *E. praecox*. This disease is generally spread by the birds picking up the infectious organism in droppings on contaminated litter or ground or by way of food or drinking water. The disease is manifested by hemorrhage, accumulation of blood in the caeca, passage of blood to the droppings, weakness and digestive disturbances. The disease often terminates in death, but the market value of fowls that survive severe infections is substantially reduced. Coccidiosis is therefore a disease of great economic importance and extensive work has been done to find new and improved methods for controlling and treating coccidial infections in poultry.

None of the 1,2,3-triazole compounds in the documents mentioned above has any anti-coccidial activity, although US Patent Specification US—A—3 577 553 discloses the anti-coccidial nature of certain 1,3,4-triazoles, viz. compounds of the formula

$$Tz — N = C(R) — Ar$$

where Tz is 1,3,4-triazol-1-yl, R is hydrogen, $C_{1-5}$ alkyl or $C_{1-5}$ haloalkyl, and Ar is a phenyl or styryl radical, optionally having one or two $C_{1-5}$ alkyl, $C_{1-5}$ alkoxy, $C_{1-5}$ alkylthio, trifluoromethyl, trifluoromethoxy, nitro or halogen substituents in the ring.

The invention is based on the discovery that certain 5-amino-1,2,3-triazoles and their substituted derivatives have a surprisingly and unexpectedly high degree of activity against coccidiosis of poultry. Certain of the compounds are novel.

The compounds used anti-coccidially in accordance with this invention have the following structural formula:

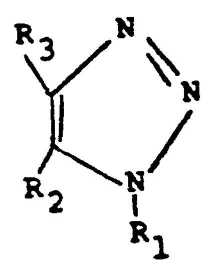

(I)

in which $R_1$ is phenyl or phenyl-($C_{1-3}$ alkyl), either unsubstituted or having from one to five halogen, cyano, trifluoromethyl, $C_{1-3}$ alkanoyl, nitro, $C_{1-3}$ alkyl, $C_{1-3}$ alkoxy, carboxy, alkyoxycarbonyl, trifluoromethoxy, acetamido, $C_{1-3}$ alkylthio, $C_{1-3}$ alkylsulphinyl, $C_{1-3}$ alkylsulphonyl, trichlorovinyl, trifluoromethylthio, trifluoromethylsulphinyl and/or trifluoromethylsulphonyl substituents; or is phenacyl, pyridyl, pyridylmethyl, naphthyl, naphthylmethyl, quinolyl or quinolylmethyl; $R_2$ is amino, $C_{1-3}$ alkylamino, di($C_{1-3}$ alkyl)amino, acetamido, acetamido, ureido, formamido, formimido or guanidino; and $R_3$ is carbamoyl, cyano, carbazoyl, amidino or N-hydroxycarbamoyl.

In the novel compounds of the present invention, $R_1$ is a benzyl radical having a trichlorovinyl substituent and none, one or two substituents as defined above, all the substituents being in the meta or para position.

Preferably, in the anticoccidial compounds $R_2$ is amino, $R_3$ is carbamoyl, and $R_1$ is phenyl or benzyl having one, two or three substituents that are halogen, cyano, trifluoromethyl, trichlorovinyl and/or methyl; especially di- or substituted phenyl or di- or tri-substituted benzyl in which the substituents are in the *meta* or *para* positions and are chloro, cyano, methyl, trifluoromethyl, or trichlorovinyl.

Examples of preferred compounds for anti-coccidial use in accordance with this invention are:

5-amino-1-(3,4-dichlorobenzyl)-1,2,3-triazole-4-carboxamide,

5-amino-1-(3,4,5-trichlorobenzyl)-1,2,3-triazole-4-carboxamide,

5-amino-1-(4-chloro-3-trifluoromethylbenzyl)-1,2,3-triazole-4-carboxamide,

5-amino-1-(4-trichlorovinylbenzyl)-1,2,3-triazole-4-carboxamide. Only the last-mentioned is novel and *per se* forms part of the present invention.

The $C_{1-3}$ alkyl groups comprise methyl, ethyl, propyl and isopropyl; the $C_{1-3}$ alkoxy groups comprise methoxy, ethoxy, propoxy, and isopropoxy; the $C_{1-3}$ alkanoyl groups comprise formyl, acetyl, and propionyl.

Administration of a small amount of at least one compound preferably by combination with poultry feed, is effective in preventing or greatly reducing the incidence of coccidiosis. The compounds are effective against both the caecal form (caused principally by *E. tenella*) and the intestinal forms (principally caused by *E. acervulina, E. brunetti, E. maxima* and *E. necatrix*). The coccidiostats of this invention are particularly effective against the species that caused caecal damage in addition to preventing the pathology caused by the coccidia. Compounds of the invention are also active against *Eimeria spp*, in other animals.

The compounds may be prepared by any one of several processes. The most general process is outlined in the following reaction scheme.

Reaction Scheme I

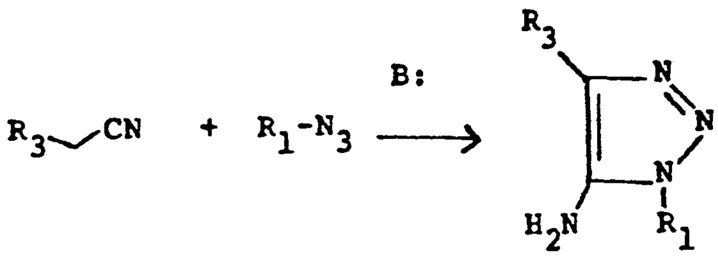


An $R_3$-methylene substituted nitrile is allowed to react with an $R_1$ azide in the presence of a base to provide the desired 5-amino-1-substituted-1,2,3-triazole. The reaction is carried out in solvents such as aromatic hydrocarbons, lower alkanols, dimethylformamide, dimethylsulphoxide or hexamethylphosphortriamide. The base may be any alkali metal or alkaline-earth metal hydroxide, alkoxide or hydride such as sodium ethoxide, potassium *t*-butoxide, magnesium ethoxide, sodium hydroxide or sodium hydride, chosen to be compatible with the reaction solvent. Generally the reaction is conducted at from −40°C to 100°C and is complete in from 15 minutes to 48 hours. The product of the reaction is isolated by known techniques.

Reaction Scheme II

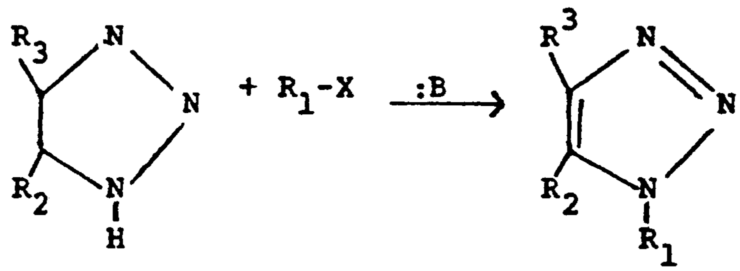


where X is a halogen, preferably chlorine or bromine. A 1-unsubstituted but otherwise appropriately substituted 1,2,3-triazole is reacted with an $R_1$ halide in the presence of a base to prepare the desired 1-substituted 1,2,3-triazole. The reaction is carried out in a solvent which may be any polar aprotic organic solvent such as dimethylformamide, dimethylsulphoxide, acetonitrile or dioxane in the presence of a base, which may be any non-nucleophilic organic or inorganic base. Suitable inorganic bases are alkali metal bases, such as sodium and potassium carbonates, phosphates, bicarbonates and hydroxides, and sodium hydride. The base is chosen for compatibility with the reaction solvent. Suitable organic bases are tertiary amines such as trialkyl-substituted amines. The reaction rate varies greatly with the nature of the proposed substituent at the $R_1$ position, the base and the solvent. Very reactive substituent and base combinations may take as little as ten minutes: at the other extreme, the reaction may take as long as two weeks. Most reactions are however complete in from 1 to 100 hours. The reaction is carried out at a temperature of from room temperature to 100°C or to the reflux temperature of the solvent system being used. The products of the reaction are isolated using known techniques.

The novel compounds of this invention are orally administered to poultry for the control and prevention of coccidiosis. Any number of conventional methods are suitable for administering the coccidiostats of this invention to poultry, for example, they may be given in the poultry feed, which is most convenient, or their drinking water. The actual quantity of the coccidiostats administered to the poultry and the concentration in the feed will vary over a wide range and be adjusted to individual needs, depending upon species of the coccidia involved and severity of the infection. The limiting criteria are that the minimum amount be sufficient to control coccidiosis and the maximum amount be such that the coccidiosis does not cause any undesirable effects.

A feed typically contains from 0.001 to 0.2 percent, preferably from 0.003 to 0.1 percent, by weight of one

of the coccidiostats of this invention. The optimum levels will naturally vary with the specific compound utilized and species of *Eimeria* involved, and can be readily determined by one skilled in the art. Levels of from 0.003 to 0.1 percent by weight of the diet are especially useful in controlling the pathology associated with *E. tenella*, as well as the intestinal dwelling species.

Depending on the compound used, levels as low as 0.001 percent possess the novel effects of reducing the number of oocysts passed in the droppings of infected chickens.

The novel coccidiostats of the invention may be readily dispersed by mechanically mixing them in finely ground form with the poultry feed, or with an intermediate formulation (premix) that is subsequently blended with other components to prepare the final poultry feed. Typical components of poultry feeds include molasses, fermentation residues, corn meal, ground and rolled oats, wheat shorts and middlings, alfalfa, clover and meat scraps, together with mineral supplements such as bone meal and calcium carbonate and vitamins.

The following non-limiting examples will serve to further illustrate the invention.

Example 1

5-Amino-1-(3,4-dichlorobenzyl)-1,2,3-triazole-4-carboxamide

Method A

A stirred mixture of 3,4-dichlorobenzyl chloride (12.6 g, 64.5 mmol) and sodium azide (7.0 g, 0.11 mole) in absolute ethanol (70 ml) was refluxed for 4.75 hours, cooled and filtered to provide a solution of 3,4-dichlorobenzyl azide. Separately, 2-cyanoacetamide (5.5 g, 65 mmol) was added to a 35°C solution of sodium (1.5 g, 65 mmol) in absolute ethanol (125 ml), and to the resulting suspension was added the above azide solution dropwise over 10 minutes. The combined mixtures were refluxed for 1 hour, kept 16 hours at ambient temperature and 1 hour at 5°C, and filtered. The crude product was dried under vacuum, dissolved in boiling ethanol (290 ml), filtered hot, and cooled to 0°C. The solid was collected by filtration and dried under vacuum to provide 12.4 g (67%) of 1-(3,4-dichlorobenzyl)-5-amino-1,2,3-triazole-4-carboxamide, m.p. 221—222°C.

Method B

A stirred, ambient temperature solution of 5-amino-1,2,3-triazole-4-carboxamide (635 mg, 5.00 mole) in dry dimethylformamide (20 ml) is treated in one portion with sodium hydride (240 mg of a 50% dispersion in mineral oil, 120 mg NaH, 5.0 mmol). After 15 min 3,4-dichlorobenzyl chloride (0.977 g, 5.00 mmol) is added. The mixture is stirred 1 hour, poured into water (20 ml), acidified to pH 6 with glacial acetic acid, and filtered. The solid is washed with water, dried, and chromatographed to provide 5-amino-1-(3,4-dichlorobenzyl)-1,2,3-triazole-4-carboxamide.

Example 2

5-Amino-1-(4-methylbenzyl)-1,2,3-triazole-4-carboxamide

A stirred mixture of 4-methylbenzyl bromide (1.3 g, 7.0 mmol) and sodium azide (754 mg, 11.6 mole) in ethanol (8 ml) was refluxed under a nitrogen atmosphere for 3 hours, cooled to ambient temperature, and filtered. Separately, 2-cyanoacetamide (588 mg, 7.0 mmol) was added to a stirred, refluxing solution of sodium (167 mg, 7.2 mmol) in ethanol (15 ml), followed by dropwise addition of the above azide solution over 20 min. The resulting slurry was refluxed 1 hour, cooled to ambient temperature, and refrigerated. The precipitate was collected by filtration, washed with ethanol, and dried under vacuum to provide 1.12 g (69%) of 1-(4-methylbenzyl)-5-amino-1,2,3-triazole-4-carboxamide, m.p. 223—225°C.

Example 3

4-Chloro-3-cyanobenzyl bromide

A mixture of 2-chloro-5-methylbenzonitrile (10.6 g, 69.9 mmol), N-bromosuccinimide (12.2 g, 68.5 mmol) and dibenzoyl peroxide (349 mg, 1.44 mmol) in benzene (350 ml) was refluxed 1.5 hours, cooled, and evaporated to dryness under vacuum. The residue was suspended in 7:3 (v/v) hexane-dichloromethane, filtered, and evaporated. The crude product was chromatographed on silica gel (650 g) eluted with 7:3 (v/v) hexane-dichloromethane to provide 4.8 g (30%) 4-chloro-3-cyanobenzyl bromide, m.p. 55—58°C.

Example 4

4-Chloro-3-cyanobenzyl azide

A stirred mixture of 4-chloro-3-cyanobenzyl bromide (3.0 g, 13 mmol), and sodium azide (1.26 mg, 19.4 mmol) was refluxed 5 hours in absolute ethanol (30 mL). The mixture was kept 18 hours at ambient temperature, filtered, and the filtrate was evaporated under vacuum. The residue was triturated with diethyl ether, filtered, and evaporated to provide 2.45 g (98%) liquid 4-chloro-3-cyanobenzyl azide; I.R. (neat): 2240, 2100 $cm^1$.

Example 5

5-Amino-1-(4-chloro-3-cyanobenzyl)-1,2,3-triazole-4-carboxamide

A stirred suspension of 2-cyanoacetamide (790 mg, 9.40 mmol) in absolute ethanol (40 ml) was treated

with sodium methoxide (495 mg, 9.16 mmol) and refluxed 10 minutes. The mixture was cooled slightly, a solution of 4-chloro-3-cyanobenzyl azide (1.35 g, 7.01 mmol) in absolute ethanol (10 ml) was added in one portion, and the mixture was refluxed 2.5 hours. The mixture was filtered hot, the solid was washed with absolute ethanol, and the combined filtrate and wash were evaporated to dryness under vacuum. The residue was triturated with diethyl ether, filtered, and washed twice with diethyl ether. The solid was recrystallized from methanol (6 ml) and dried at 65°C under vacuum to provide 635 mg (24%) 5-amino-1-(4-chloro-3-cyanobenzyl)-1,2,3-triazole-4-carboxamide, m.p. 172—175°C.

Example 6

1-(3,4-Dichlorobenzyl)-5-methylamino-1,2,3-triazole-4-carboxamide

A mixture of 5-amino-1-(3,4-dichlorobenzyl)-1,2,3-triazole-4-carboxamide (2.66 g, 10.0 mmol), methyl iodide (1.42 g, 10.0 mmol), and potassium carbonate (1.38 g, 10.0 mmol) in *N,N*-dimethylformamide (20 ml) is stirred 48 hours at ambient temperature, poured into water (150 ml), and filtered. Chromatography provides 1-(3,4-dichlorobenzyl)-5-methylamino-1,2,3-triazole-4-carboxamide.

Example 7

5-Amino-1-(4-chloro-3-cyanobenzyl)-1,2,3-triazole-4-carboxamide

A stirred suspension of 2-cyanoacetamide (790 mg, 9.4 mmol) in absolute ethanol (40 ml) was treated with sodium methoxide (495 mg, 9.2 mmol) and refluxed 10 minutes. The mixture was cooled lightly, a solution of 4-chloro-3-cyanobenzyl azide (1.35 g, 7.0 mmol) in absolute ethanol was added, and the mixture was refluxed 2.5 hours. The mixture was filtered hot, and the filtrate evaporated under vacuum. The residue was triturated with diethyl ether, filtered, and washed twice with diethyl ether. The solid was recrystallized from refluxing methanol, filtered, washed twice with methanol and once with diethyl ether, and dried at 65°C under vacuum to provide 635 mg (33%) 5-amino-1-(4-chloro-3-cyanobenzyl)-1,2,3-triazole-4-carboxamide, m.p. 172—175°C.

Example 8

5-Amino-1-(4-cyano-3,5-dichlorobenzyl)-1,2,3-triazole-4-carboxamide

A stirred, ambient temperature solution of 5-amino-1,2,3-triazole-4-carboxamide (630 mg, 5.0 mmol) in dry *N,N*-dimethylformamide (20 ml) was treated with sodium hydride (250 mg of a 50% dispersion in mineral oil, 125 mg NaH, 5.2 mmol). The resulting suspension was stirred 10 min., 4-cyano-3,5-dichlorobenzyl chloride (1.1 g, 5.0 mmol) was added, and the mixture was stirred 2 hours. The reaction was quenched by pouring into ice and water (80 ml). The suspension was filtered and washed three times with water. The solid was suspended in 19:1 (v/v) dichloromethane-methanol and filtered to provide 364 mg (23%) 5-amino-1-(4-cyano-3,5-dichlorobenzyl)-1,2,3-triazole-4-carboxamide. Recrystallization from ethanol provided material of m.p. 238—239.5°C.

**Claims**

1. A compound having the formula:

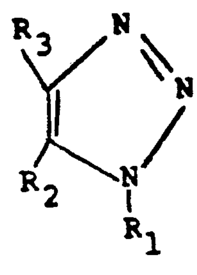

in which $R_1$ is a benzyl radical having a trichlorovinyl substituent and none, one or two halogen, cyano, trifluoromethyl, $C_{1-3}$ alkanoyl, nitro, $C_{1-3}$ alkyl, $C_{1-3}$ alkoxy, carboxy, alkoxycarbonyl, trifluoromethoxy, acetamido, $C_{1-3}$ alkylthio, $C_{1-3}$ alkylsulphinyl, $C_{1-3}$ alkylsulphonyl, trichlorovinyl, trifluoromethylthio, trifluoromethylsulphinyl and/or trifluoromethylsulphonyl substituents; $R_2$ is amino, $C_{1-3}$ alkylamino, d($C_{1-3}$ alkyl)amino, acetamido, ureido, formamido, formimido or guanidino; and $R_3$ is carbamoyl, cyano, carbazoyl, amidino or N-hydroxycarbamoyl.

2. A compound as claimed in Claim 1 in which $R_1$ is a benzyl radical having a trichlorovinyl substituent and none, one or two substituents that are halogen, cyano, trifluoromethyl, trichlorovinyl and/or methyl; $R_2$ is amino and $R_3$ is carbamoyl.

3. 5-amino-1-(4-trichlorovinylbenzyl)-1,2,3-triazole-4-carboxamide.

4. A method of preparing a compound as claimed in Claim 1 in which $R_2$ is amino by reaction of a monosubstituted acetonitrile $R_3CH_2CN$ with an azide $R_1N_3$ in the presence of an alkali metal or alkaline-earth metal hydroxide, alkoxide or hydride in a compatible solvent.

5. A method as claimed in claim 4 in which the monosubstituted acetonitrile is 2-cyanoacetamide, malononitrile, or a lower alkyl ester of cyanoacetic acid.

6. A method of preparing a compound as claimed in Claim 1 comprising reacting a compound as claimed in Claim 1 except that $R_1$ is hydrogen with an $R_1$ halide in the presence of a non-nucleophilic organic or inorganic base and in a compatible polar aprotic organic solvent.

7. A method of preparing a compound as claimed in Claim 1 in which $R_2$ is $C_{1-3}$ alkyl amino or di($C_{1-3}$ alkyl)amino comprising reacting a compound as claimed in Claim 1 in which $R_2$ is amino with a $C_{1-3}$ alkyl halide in the presence of a non-nucleophilic organic or inorganic base and in a compatible polar aprotic organic solvent.

8. A compound having the formula

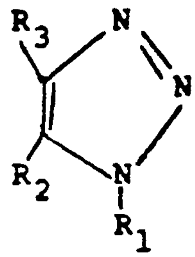

in which $R_1$ is phenyl or phenyl-($C_{1-3}$alkyl), either unsubstituted or having from one to five halogen, cyano, trifluoromethyl, $C_{1-3}$ alkanoyl, nitro, $C_{1-3}$ alkyl, $C_{1-3}$ alkoxy, carboxy, alkoxycarbonyl, trifluoromethoxy, acetamido, $C_{1-3}$ alkylthio, $C_{1-3}$ alkylsulphinyl, $C_{1-3}$ alkylsulphonyl, trichlorovinyl, trifluoromethylthio, trifluoromethylsulphinyl and/or trifluoromethylsulphonyl substituents; or is phenacyl, pyridyl, pyridylmethyl, naphthyl, naphthylmethyl, quinolyl or quinolylmethyl; $R_2$ is amino, $C_{1-3}$ alkylamino, di($C_{1-3}$ alkyl) amino, acetamido, ureido, formamido, formimido or guanidino; and $R_3$ is carbamoyl, cyano, carbazoyl, amidino or N-hydroxycarbamoyl, for use in the prevention or treatment of coccidiosis.

9. A composition useful for the prevention and treatment of coccidiosis which comprises an inert carrier and a compound having the formula set forth in Claim 8.

**Patentansprüche**

1. Verbindung mit der Formel

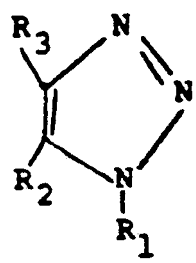

worin $R_1$ ein Benzylrest mit einem Trichlorvinylsubstituenten und keinem, einem oder zwei Halogen-, Cyano-, Trifluormethyl-, $C_{1-3}$-Alkanoyl-, Nitro-, $C_{1-3}$-Alkyl-, $C_{1-3}$-Alkoxy-, Carboxy-, Alkoxycarbonyl-, Trifluormethoxy-, Acetamido-, $C_{1-3}$-Alkylthio-, $C_{1-3}$-Alkylsulfinyl-, $C_{1-3}$-Alkylsulfonyl-, Trichlorvinyl-, Trifluormethylthio-, Trifluormethylsulfinyl- und/oder Trifluormethylsulfonylsubstituenten ist; $R_2$ Amino, $C_{1-3}$-Alkylamino, Di($C_{1-3}$-alkyl)amino, Acetamido, Ureido, Formamido, Formimido oder Guanidino ist; und $R_3$ Carbamoyl, Cyano, Carbazoyl, Amidino oder N-Hydroxycarbamoyl ist.

2. Verbindung, wie in Anspruch 1 beansprucht, worin $R_1$ ein Benzylrest mit einem Trichlorvinylsubstituenten und keinem, einem oder zwei Substituenten ist, die Halogen, Cyano, Trilfluormethyl, Trichlorvinyl und/oder Methyl sind; $R_2$ Amino ist und $R_3$ Carbamoyl ist.

3. 5-Amino-1-(4-trichlorvinylbenzyl)-1,2,3-triazol-4-carboxamid.

4. Verfahren zur Herstellung einer Verbindung, wie in Anspruch 1 beansprucht, worin $R_2$ Amino ist, durch Reaktion eines monosubstituierten Acetonitrils $R_3CH_2CN$ mit einem Azid $R_1N_3$ in Gegenwert eines Alkalimetall- oder Erdalkalimetallhydroxids, -alkoxids oder -hydrids in einem passenden Lösungsmittel.

5. Verfahren, wie in Anspruch 1 beansprucht, worin das monosubstituierte Acetonitril 2-Cyanoacetamid, Malononitril oder ein Niederalkylester von Cyanessigsäure ist.

6. Verfahren zur Herstellung einer Verbindung, wie in Anspruch 1 beansprucht, durch Umsetzung einer Verbindung, wie in Anspruch 1 beansprucht, außer daß $R_1$ Wasserstoff ist, mit einem $R_1$-Halogenid in Gegenwart einer nicht nukleophilen organischen oder anorganischen Base in einem passenden polaren aprotischen, organischen Lösungsmittel.

7. Verfahren zur Herstellung einer Verbindung, wie in Anspruch 1 beansprucht, worin $R_2$ $C_{1-3}$-

Alkylamido oder Di($C_{1-3}$-alkyl)amino ist, durch Umsetzung einer Verbindung, wie in Anspruch 1 beansprucht, worin $R_2$ Amino ist, mit einem $C_{1-3}$-Alkylhalogenid in Gegenwart einer nicht nukleophilen organischen oder anorganischen Base in einem passenden, polaren aprotischen, organischen Lösungsmittel.

8. Verbindung mit der Formel

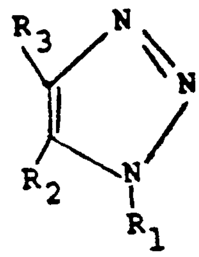

worin $R_1$ Phenyl oder Phenyl-($C_{1-3}$-alkyl) ist, entweder unsubstituiert oder mit einem bis fünf Halogen-, Cyano-, Trifluormethyl-, $C_{1-3}$-Alkanoyl-, Nitro-, $C_{1-3}$-Alkyl-, $C_{1-3}$-Alkoxy-, Carboxy-, Alkoxycarbonyl-, Trifluormethoxy-, Acetamido-, $C_{1-3}$-Alkylthio-, $C_{1-3}$-Alkylsulfinyl-, $C_{1-3}$-Alkylsulfonyl-, Trichlorvinyl-, Trifluormethylthio-, Trifluormethylsulfinyl- und/oder Trifluormethylsulfonylsubstituenten; oder Phenacyl, Pyridyl, Pyridylmethyl, Naphthyl, Naphthylmethyl, Chinolyl oder Chinolylmethyl ist; $R_2$ Amino, $C_{1-3}$-Alkylamino, Di($C_{1-3}$-alkyl)amino, Acetamido, Ureido, Formamido, Formimido oder Guanidino ist; und $R_3$ Carbamoyl, Cyano, Carbazoyl, Amidino oder N-Hydroxycarbamoyl ist, zur Verwendung bei der Vorbeugung oder Behandlung von Kokzidiose.

9. Zusammensetzung, geeignet zur Vorbeugung und Behandlung von Kokzidiose, welche einen inerten Träger und eine Verbindung mit der in Anspruch 8 bezeichneten Formel umfaßt.

**Revendications**

1. Composé ayant pour formule:

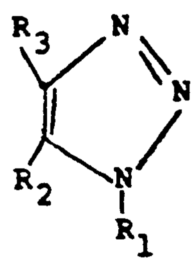

dans laquelle $R_1$ est un radical benzyle ayant un substituant trichlorovinyle et aucun, un ou deux substituants halogènes, cyano, trifluorométhyles, alcanoyles en $C_{1-3}$, nitro, alkyles en $C_{1-3}$, alcoxy en $C_{1-3}$, carboxy, alcoxycarbonyles, trifluorométhoxy, acétamido, alkylthio en $C_{1-3}$, alkylsulfinyles en $C_{1-3}$, alkylsulfonyles en $C_{1-3}$, trichlorovinyles, trifluorométhylthio, trifluorométhylsulfinyles et/ou trifluorométhylsulfonyles; $R_2$ est un amino, un alkylamino en $C_{1-3}$, und di(alkyl en $C_{1-3}$)amino, un acétamido, un uréido, un formamido, un formamido ou un guanidino; et $R_3$ est un carbamoyle, un cyano, un carbazoyle, un amidino ou un N-hydroxycarbamoyle.

2. Composé selon la revendication 1, où $R_1$ est un radical benzyle ayant un substituant trichlorovinyle et aucun, un ou deux substituants choisis parmi un halogène, un cyano, un trifluorométhyle, un trichlorovinyle et/ou un méthyle; $R_2$ est un amino et $R_3$ est un carbamoyle.

3. 5-amino-1-(4-trichlorovinylbenzyl)-1,2,3-triazole-4-carboxamide.

4. Procédé pour préparer un composé selon la revendication 1 où $R_2$ est un amino par réaction d'un acétonitrile monosubstitué $R_3CH_2CN$ avec un azide $R_1N_3$ en présence d'un hydroxyde, alcoolate ou hydrure de métal alcalin ou de métal alcalino-terreux dans un solvant compatible.

5. Procédé selon la revendication 4, dans lequel l'acétonitrile monosubstitué est le 2-cyanoacétamide, le malononitrile ou un ester d'alkyle inférieur de l'acide cyanoacétique.

6. Procédé pour préparer un composé selon la revendication 1 comprenant la réaction d'un composé selon la revendication 1, si ce n'est que $R_1$ est un hydrogène, avec un halogénure de $R_1$ en présence d'une base organique ou minérale non nucléophile et dans un solvant organique aprotique polaire compatible.

7. Procédé pour préparer un composé selon la revendication 1, où $R_2$ est un alkylamino en $C_{1-3}$ ou un di(alkyl en $C_{1-3}$)amino, comprenant la réaction d'un composé selon la revendication 1 où $R_2$ est un amino avec un halogénure d'alkyle en $C_{1-3}$ en présence d'une base organique ou minérale non nucléophile et dans un solvant organique aprotique polaire compatible.

8. Composé répondant à la formule

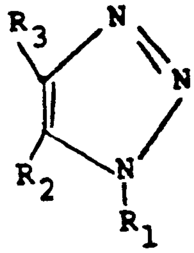

dans laquelle $R_1$ est un phényle ou un phényl-(alkyle en $C_{1-3}$) soit non substitué, soit ayant de 1 à 5 substituants halogènes, cyano, trifluorométhyles, alcanoyles en $C_{1-3}$, nitro, alkyles en $C_{1-3}$, alcoxy en $C_{1-3}$, carboxy, alcoxycarbonyles, trifluorométhoxy, acétamido, alkylthio en $C_{1-3}$, alkylsulfinyles en $C_{1-3}$, alkylsulfonyles en $C_{1-3}$, trichlorovinyles, trifluorométhylthio, trifluorométhylsulfinyles et/ou trifluorométhylsulfonyles; ou est un phénacyle, pyridyle, pyridylméthyl, naphtyle, naphtylméthyle, quinolyle ou quinolylméthyle; $R_2$ est un amino, un alkylamino en $C_{1-3}$, un di(alkyl en $C_{1-3}$)amino, un acétamido, un uréido, un formamido, un formamido ou un guanidino; et $R_3$ est un carbamoyle, un cyano, un carbazoyle, un amidino ou un N-hydroxycarbamoyle, pour l'utilisation dans la prévention ou le traitement de la coccidiose.

9. Composition utile pour la prévention et le traitement de la coccidiose, qui comprend un support inerte et un composé répondant à la formule indiquée dans la revendication 8.